Europäisches Patentamt

European Patent Office

Office européen des brevets

(19)

(11) **EP 1 568 309 A1**

(12) **EUROPEAN PATENT APPLICATION**

(43) Date of publication:
**31.08.2005 Bulletin 2005/35**

(51) Int Cl.⁷: **A61B 5/00**, G01K 17/00

(21) Application number: **04007593.9**

(22) Date of filing: **29.03.2004**

(84) Designated Contracting States:
**AT BE BG CH CY CZ DE DK EE ES FI FR GB GR HU IE IT LI LU MC NL PL PT RO SE SI SK TR**
Designated Extension States:
**AL LT LV MK**

(30) Priority: **24.02.2004 JP 2004048546**

(71) Applicant: **Hitachi, Ltd.**
**Tokyo 100-8010 (JP)**

(72) Inventors:
• **Cho, Ok-Oyung Hitachi Ltd. Intel. Prop. Grp.**
**Chiyoda-ku Tokyo 100-8220 (JP)**

• **Kim, Yoon-Ok Hitachi Ltd. Intel. Prop. Grp.**
**Chiyoda-ku Tokyo 100-8220 (JP)**
• **Ichige,Yukiko Hitachi Ltd. Intel. Prop. Grp.**
**Chiyoda-ku Tokyo 100-8220 (JP)**
• **Mitsumaki,Hiroshi Hitachi,Lt.Intel.Prop.Grp.**
**Chiyoda-ku Tokyo 100-8220 (JP)**

(74) Representative: **Strehl Schübel-Hopf & Partner**
**Maximilianstrasse 54**
**80538 München (DE)**

(54) **Blood sugar level measuring apparatus**

(57)     Blood sugar levels are measured non-invasively based on temperature measurement. Non-invasively measured blood sugar level values obtained by a temperature measurement scheme are corrected by blood oxygen saturation and blood flow volume, thereby stabilizing the measurement data.

FIG. 5

EP 1 568 309 A1

**Description**

CLAIM OF PRIORITY

The present application claims priority from Japanese application JP 2004-048546 filed on February 24, 2004, the content of which is hereby incorporated by reference to this application.

BACKGROUND OF THE INVENTION

Field of the Invention

The present invention relates to a non-invasive blood sugar level measuring apparatus for measuring glucose concentration in a living body without blood sampling.

Background Art

Hilson *et al.* report facial and sublingual temperature changes in diabetics following intravenous glucose injection (Non-Patent Document 1). Scott *et al.* discuss the issue of diabetics and thermoregulation (Non-Patent Document 2). Based on such researches, Cho *et al.* suggests a method and apparatus for determining blood glucose concentration by temperature measurement without requiring the collection of a blood sample (Patent Documents 1 and 2).
Various other attempts have been made to determine glucose concentration without blood sampling. For example, a method has been suggested (Patent Document 3) whereby a measurement site is irradiated with near-infrared light of three wavelengths, and the intensity of transmitted light as well as the temperature of the living body is detected. Then, a representative value of the second-order differentiated values of absorbance is calculated, and the representative value is corrected in accordance with the difference between the living body temperature and a predetermined reference temperature. A blood sugar level corresponding to the thus corrected representative value is then determined. An apparatus is also provided (Patent Document 4) whereby a measurement site is heated or cooled while monitoring the living body temperature. The degree of attenuation of light based on light irradiation is measured at the moment of temperature change so that the glucose concentration responsible for the temperature-dependency of the degree of light attenuation can be measured. Further, an apparatus is reported (Patent Document 5) whereby an output ratio between reference light and the light transmitted by an irradiated sample is taken, and then a glucose concentration is calculated by a linear expression of the logarithm of the output ratio and the living body temperature.
[Non-Patent Document 1] R.M. Hilson and T.D.R. Hockaday, "Facial and sublingual temperature changes following intravenous glucose injection in diabetics," Diabete & Metabolisme, 8, pp.15-19: 1982
[Non-Patent Document 2] A.R. Scott, T. Bennett, I.A. MacDonald, "Diabetes mellitus and thermoregulation," Can. J. Physiol. Pharmacol., 65, pp. 1365-1376: 1987
[Patent Document 1] U.S. Patent No. 5,924,996
[Patent Document 2] U.S. Patent No. 5,795,305
[Patent Document 3] JP Patent Publication (Kokai) No. 2000-258343 A
[Patent Document 4] JP Patent Publication (Kokai) No. 10-33512 A (1998)
[Patent Document 5] JP Patent Publication (Kokai) No. 10-108857 A (1998)

SUMMARY OF THE INVENTION

Glucose (blood sugar) in blood is used for glucose oxidation reaction in cells to produce necessary energy for the maintenance of a living body. In the basal metabolism state, in particular, most of the produced energy is converted into heat energy for the maintenance of body temperature. Thus, it can be expected that there is some relationship between blood glucose concentration and body temperature. However, as is evident from the way sicknesses cause fever, the body temperature also varies due to factors other than blood glucose concentration. While methods have been proposed to determine blood glucose concentration by temperature measurement without blood sampling, they lack sufficient accuracy.
It is the object of the invention to provide a method and apparatus for determining blood glucose concentration with high accuracy based on temperature data of a subject without blood sampling.
Blood sugar is delivered to the cells throughout the human body via the blood vessel system, particularly the capillary blood vessels. In the human body, complex metabolic pathways exist. Glucose oxidation is a reaction in which, fundamentally, blood sugar reacts with oxygen to produce water, carbon dioxide, and energy. Oxygen herein refers to the oxygen delivered to the cells via blood. The amount of oxygen supply is determined by the blood hemoglobin concentration, the hemoglobin oxygen saturation, and the volume of blood flow. On the other hand, the heat produced in the body by glucose oxidation is dissipated from the body by convection, heat radiation, conduction, and so on. On the assumption that the body temperature is determined by the balance between the amount of energy produced in the body by glucose burning, namely heat production, and heat dissipation such as mentioned above, we set up the following model:

(1) The amount of heat production and the amount of heat dissipation are considered equal.
(2) The amount of heat production is a function of the blood glucose concentration and the amount of oxygen supply.
(3) The amount of oxygen supply is determined by the blood hemoglobin concentration, the blood hemoglobin oxygen saturation, and the volume of blood flow in the capillary blood vessels.
(4) The amount of heat dissipation is mainly determined by heat convection and heat radiation.

The inventors have achieved the present invention after realizing that blood sugar levels can be accurately determined on the basis of the results of measuring the temperature of the body surface and parameters relating to oxygen concentration in blood and blood flow volume, in accordance with the aforementioned model. The parameters can be measured from a part of the human body, such as the fingertip. Parameters relating to convection and radiation can be determined by carrying out thermal measurements on the fingertip. Parameters relating to blood hemoglobin concentration and blood hemoglobin oxygen saturation can be obtained by spectroscopically measuring blood hemoglobin and determining the ratio of hemoglobin bound with oxygen to hemoglobin not bound with oxygen. With regard to the parameters relating to blood hemoglobin concentration and blood hemoglobin oxygen saturation , measurement accuracy would not be significantly lowered if pre-stored constants are employed rather than taking measurements. The parameter relating to the volume of blood flow can be determined by measuring the amount of heat transfer from the skin.

In one aspect, the invention provides a blood sugar level measuring apparatus comprising:

a heat amount measurement portion for measuring a plurality of temperatures derived from a body surface and obtaining information used for calculating the amount of heat transferred by convection and the amount of heat transferred by radiation, both related to the dissipation of heat from said body surface;
an oxygen level measuring portion for obtaining information about blood oxygen level;
a storage portion for storing a relationship between parameters corresponding to said plurality of temperatures and blood oxygen level and blood sugar levels;
a calculating portion which converts a plurality of measurement values fed from said heat amount measuring portion and said oxygen level measurement portion into said parameters, and computes a blood sugar level by applying said parameters to said relationship stored in said storage portion;
a display portion for displaying the blood sugar level calculated by said calculating portion;
a plurality of operation buttons including a measurement start button for instructing the start of a measurement, and control buttons for performing controls other than the instruction for starting a measurement; and
a button signal processing filter mechanism for processing an input signal from said operation buttons, wherein:

said oxygen level measurement portion includes a blood flow volume measurement portion for obtaining information about blood flow volume, and an optical measurement portion for obtaining blood hemoglobin concentration and hemoglobin oxygen saturation, wherein said blood flow volume measurement portion includes:

a body-surface contact portion;
an adjacent temperature detector disposed adjacent to said body-surface contact portion;
an indirect temperature detector for detecting the concentration at a position spaced apart from said body-surface contact portion; and
a heat conducting member connecting said body-surface contact portion and said indirect temperature detector.

In another aspect, the invention provides a blood sugar level measuring apparatus comprising:

an ambient temperature measuring device for measuring ambient temperature;
a body-surface contact portion to which a body surface is brought into contact;
a radiant heat detector for measuring radiant heat from said body surface;
a heat conducting member disposed in contact with said body-surface contact portion;
an adjacent temperature detector disposed adjacent to said body-surface contact portion;
an indirect temperature detector disposed at a position that is adjacent to said heat conducting member and that is spaced apart from said body-surface contact portion, said indirect temperature detector measuring temperature at the position spaced apart from said body-surface contact portion;

a light source for irradiating said body-surface contact portion light with at least two different wavelengths;

a light detector for detecting reflected light produced as said light is reflected by said body surface;

a converter for converting outputs from said adjacent temperature detector, said indirect temperature detector, said ambient temperature detector, said radiant temperature detector and said light detector, into parameters;

a calculating portion in which a relationship between said parameters and blood sugar levels is stored in advance, and which calculates a blood sugar level by applying said parameters to said relationship;

a display for displaying the blood sugar level outputted from said calculating portion;

a plurality of operation buttons including a measurement start button for instructing the start of a measurement, and control buttons for performing controls other than the instruction for starting a measurement; and

a button signal processing filter mechanism for processing an input signal from operation buttons.

In yet another aspect, the invention provides a blood sugar level measuring apparatus comprising:

an ambient temperature measuring device for measuring ambient temperature;

a body-surface contact portion to which a body surface is brought into contact;

a radiant heat detector for measuring radiant heat from said body surface;

a heat conducting member disposed in contact with said body-surface contact portion;

an adjacent temperature detector disposed adjacent to said body-surface contact portion;

an indirect temperature detector disposed at a position that is adjacent to said heat conducting member and that is spaced apart from said body-surface contact portion, said indirect temperature detector measuring temperature at the position spaced apart from said body-surface contact portion;

a storage portion where information about blood hemoglobin concentration and blood hemoglobin oxygen saturation is stored;

a converter for converting outputs from said adjacent temperature detector, said indirect temperature detector, said ambient temperature measuring device and said radiant heat detector, into a plurality of parameters;

a calculating portion in which a relationship between said parameters and blood sugar levels is stored, said calculating portion including a processing portion for calculating a blood sugar level by applying said parameters to said relationship;

a display for displaying the blood sugar level outputted from said calculating portion;

a plurality of operation buttons including a measurement start button for instructing the start of a measurement, and control buttons for performing controls other than the instruction for starting a measurement; and

a button signal processing filter mechanism for processing an input signal from said operation buttons.

The button signal processing filter mechanism is adapted to invalidate an input signal from the operation buttons other than the measurement start button when the apparatus is in a measurement standby state, and invalidate an input signal from all of the operation buttons during measurement. The measurement start button may also serve as a power button of the apparatus.

In accordance with the invention, blood sugar levels can be determined in an non-invasive measurement with the same level of accuracy with that of the conventional invasive methods.

BRIEF DESCRIPTION OF THE DRAWINGS

Fig. 1 shows a model of the transfer of heat from a body surface to a block.

Fig. 2 shows changes in measurement values of temperatures $T_1$ and $T_2$ with time.

Fig. 3 shows an example of the measurement of a change in temperature $T_3$ with time.

Fig. 4 shows the relationship between measurement values obtained by various sensors and parameters derived therefrom.

Fig. 5 shows a top plan view and a lateral cross section of a non-invasive blood sugar level measuring apparatus according to the present invention.

Fig. 6 shows the flow of operation involving the finger.

Fig. 7 shows the flow of operation of the apparatus in response to button inputs.

Fig. 8 shows the details of a measurement portion.

Fig. 9 is a conceptual chart illustrating the flow of data processing in the apparatus.

Fig. 10 schematically shows an example of the structure inside the apparatus.

Fig. 11 shows the plots of the glucose concentration value calculated by the invention and the glucose concentration value measured by the enzyme electrode method.

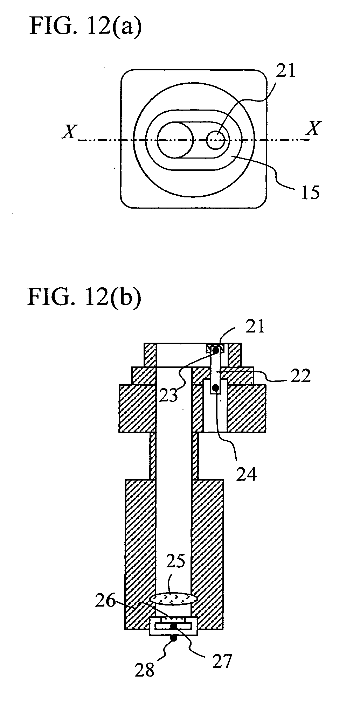

Fig. 12 shows the details of another example of the measurement portion.

Fig. 13 is a conceptual chart illustrating the location where data is stored in the apparatus.

Fig. 14 shows the plots of the glucose concentration value calculated by the invention and the glucose concentration value measured by the enzyme electrode method.

## DESCRIPTION OF THE PREFERRED EMBODIMENTS

The invention will now be described by way of preferred embodiments thereof with reference made to the drawings.

Initially, the above-mentioned model will be described in more specific terms. Regarding the amount of heat dissipation, convective heat transfer, which is one of the main causes of heat dissipation, is related to temperature difference between the ambient (room) temperature and the body-surface temperature. The amount of heat dissipation due to radiation, another main cause of dissipation, is proportional to the fourth power of the body-surface temperature according to the Stefan-Boltzmann law. Thus, it can be seen that the amount of heat dissipation from the human body is related to the room temperature and the body-surface temperature. Another major factor related to the amount of heat production, the oxygen supply volume, is expressed as the product of hemoglobin concentration, hemoglobin oxygen saturation, and blood flow volume.

The hemoglobin concentration can be measured based on the absorbance of light at the wavelength (iso-absorption wavelength) at which the molar absorption coefficient of the oxygen-bound hemoglobin and that of the reduced (deoxygenated) hemoglobin are equal. The hemoglobin oxygen saturation can be measured by measuring the absorbance of the iso-absorption wavelength and at least one other wavelength at which the ratio of the molar absorption coefficient of the oxygen-bound hemoglobin to that of the reduced (deoxygenated) hemoglobin is known, and then solving simultaneous equations. Thus, the hemoglobin concentration and the hemoglobin oxygen saturation can be obtained by measuring absorbance at at least two wavelengths.

The rest is the blood flow volume, which can be measured by various methods. One example will be described below.

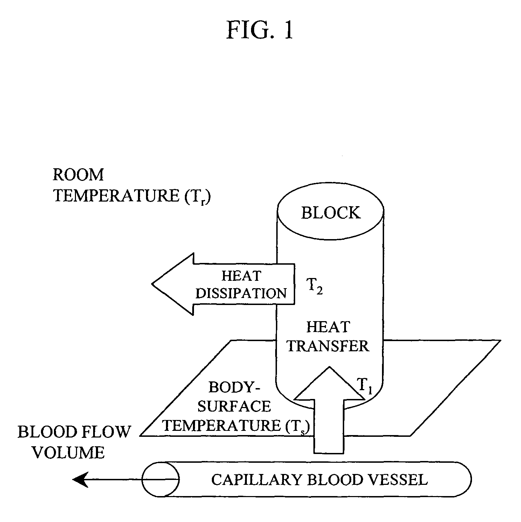

Fig. 1 shows a model for the description of the transfer of heat from the body surface to a solid block with a certain heat capacity as the block is brought into contact with the body surface for a certain time and then separated. The block is made of resin such as plastic or vinyl chloride. In the illustrated example, attention will be focused on the chronological variation of a temperature $T_1$ of a portion of the block in contact with the body surface, and the chronological variation of a temperature $T_2$ at a point on the block away from the body surface. The blood flow volume can be estimated by monitoring mainly the chronological variation of the temperature $T_2$ (at the spatially distant point on the block). The details will be described later.

Before the block comes into contact with the body surface, the temperatures $T_1$ and $T_2$ at the two points of the block are equal to the room temperature $T_r$. When a body-surface temperature $T_s$ is higher than the room temperature $T_r$, the temperature $T_1$ swiftly rises as the block comes into contact with the body surface, due to the transfer of heat from the skin, and it approaches the body-surface temperature $T_s$. On the other hand, the temperature $T_2$, which is lower than the temperature $T_1$ due to the dissipation of the heat conducted through the block from its surface, rises more gradually than the temperature $T_1$. The chronological variation of the temperatures $T_1$ and $T_2$ depends on the amount of heat transferred from the body surface to the block, which in turn depends on the blood flow volume in the capillary blood vessels under the skin. If the capillary blood vessels are regarded as a heat exchanger, the coefficient of heat transfer from the capillary blood vessels to the surrounding cell tissues is given as a function of the blood flow volume. Thus, by measuring the amount of heat transfer from the body surface to the block by monitoring the chronological variation of the temperatures $T_1$ and $T_2$, the amount of heat transmitted from the capillary blood vessels to the cell tissues can be estimated, which in turn makes it possible to estimate the blood flow volume.

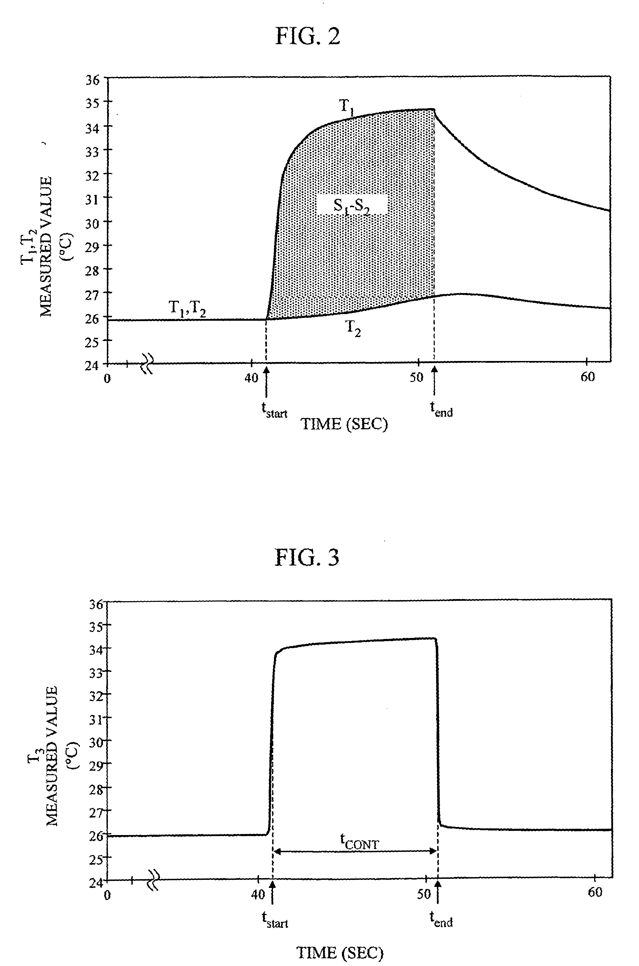

Fig. 2 shows the chronological variation of the measured values of the temperature $T_1$ at the portion of the block in contact with the body surface and the temperature $T_2$ at the point on the block away from the body-surface contact position. As the block comes into contact with the body surface, $T_1$ swiftly rises, and it gradually drops as the block is brought out of contact.

Fig. 3 shows the chronological variation of the measured value of a temperature $T_3$ measured by a radiation temperature detector. As the temperature $T_3$ measured is that due to the radiation from the body surface, this sensor can more sensitively react to temperature changes than other sensors. Because radiation heat propagates as an electromagnetic wave, it can transmit temperature changes instantaneously.

Then, the $T_1$ measured value between $t_{start}$ and $t_{end}$ is approximated by an S curve, such as a logistic curve. A logistic curve is expressed by the following equation:

$$T = \frac{b}{1 + c \times \exp(-a \times t)} + d$$

where T is temperature, and t is time.

The measured value can be approximated by determining factors a, b, c, and d by the non-linear least-squares method. For the resultant approximate expression, T is integrated between time $t_{start}$ and time $t_{end}$ to obtain a value $S_1$.

Similarly, an integrated value $S_2$ is calculated from the $T_2$ measured value. The smaller the ($S_1$ - $S_2$) is, the larger the amount of transfer of heat from the finger surface to the position of $T_2$. ($S_1$ - $S_2$) becomes larger with increasing finger contact time $t_{cont}$ (=$t_{end}$ - $t_{start}$). Thus, $e_5/(t_{cont} \times (S_1 - S_2))$ is designated as a parameter $X_5$ indicating the volume of blood flow, where $e_3$ is a proportionality coefficient.

It will be seen from the above description that the measured quantities necessary for the determination of blood glucose concentration by the aforementioned model are the room temperature (ambient temperature), body surface temperature, temperature changes in the block in contact with the body surface, the temperature due to radiation from the body surface, and the absorbance of at least two wavelengths.

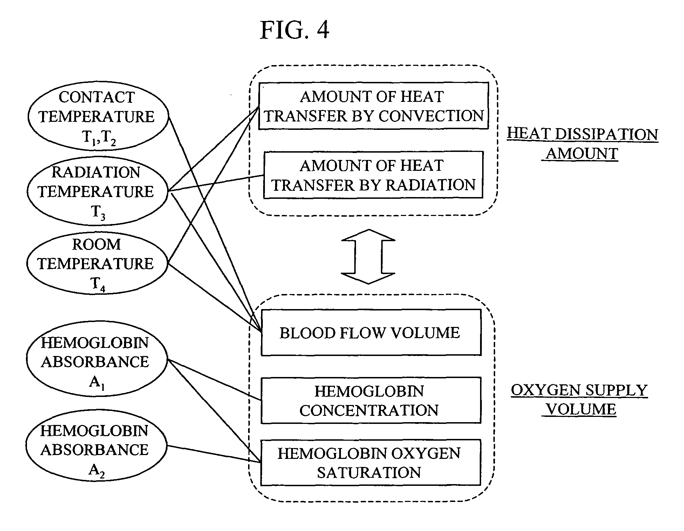

Fig. 4 shows the relationships between the measured values provided by various sensors and the parameters derived therefrom. A block is brought into contact with the body surface, and chronological changes in the two kinds of temperatures $T_1$ and $T_2$ are measured by two temperature sensors provided at two locations of the block. Separately, the radiation temperature $T_3$ on the body surface and the room temperature $T_4$ are measured. Absorbance A1 and A2 are measured at at least two wavelengths related to the absorption of hemoglobin. The temperatures $T_1$, $T_2$, $T_3$, and $T_4$ provide parameters related to the volume of blood flow. The temperature $T_3$ provides a parameter related to the amount of heat transferred by radiation. The temperatures $T_3$ and $T_4$ provide parameters related to the amount of heat transferred by convection. Absorbance $A_1$ provides a parameter relating to hemoglobin concentration. Absorbance $A_1$ and $A_2$ provide parameters relating to hemoglobin oxygen saturation.

By observing the temperature change of $T_1$ in time, the timing at which the body surface comes into contact with the block is detected. The amount of temperature change is calculated, and if the change amount is within a certain threshold value, it is determined that the body surface has made a contact. The change amount is proportional to the amount of heat transferred from the body surface to the block. As the heat is produced by the blood, the greater the blood flow volume of a person, the larger the slope, and the smaller the blood flow volume, the smaller the change amount. However, there is a certain range of blood flow volume that man can take, and therefore there is also a certain range of the change amount. For example, the blood flow volume increases after exercise, while it decreases at rest.

On the other hand, by observing the temperature changes in $T_3$, the timing at which the body surface leaves the block is detected. $T_1$ cannot provide an accurate reading of the temperature change due to the fact that the heat remains in the block even after the body surface is separated. Thus, by using T3, the timing of the leaving of the body surface can be more accurately determined.

Hereafter, an example of the apparatus for non-invasively measuring blood sugar levels according to the principle of the invention will be described.

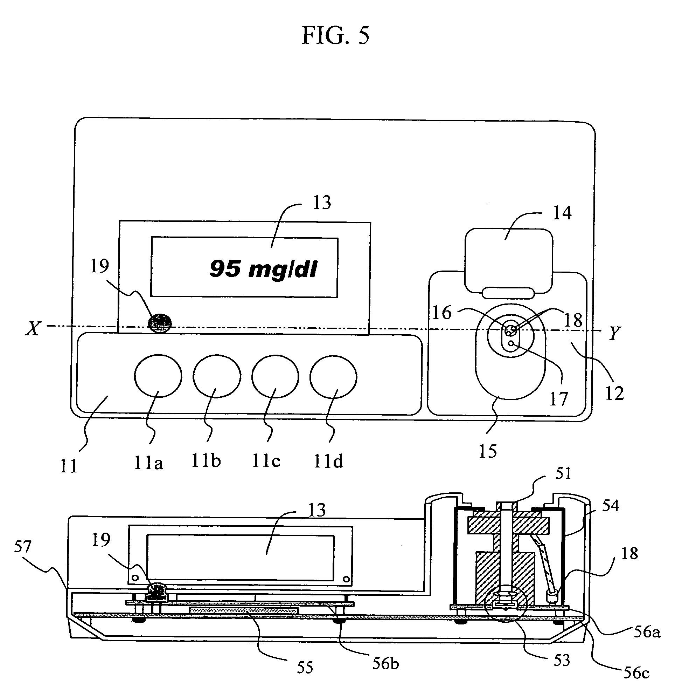

Fig. 5 shows a top plan view and a lateral cross section of the non-invasive blood sugar level measuring apparatus according to the invention. While in this example the skin on the ball of the fingertip is used as the body surface, other parts of the body surface may be used.

On the upper surface of the apparatus are provided an operating portion 11, a measurement portion 12 where the finger to be measured is to be placed, and a display portion 13 for displaying the result of measurement, the state of the apparatus, measured values, and so on. The operating portion 11 includes four push buttons 11 a to 11d for operating the apparatus. One of the buttons, such as button 11d, is a measurement start button. The measurement portion 12 has a cover 14 which, when opened (as shown), reveals a finger rest portion 15 with an oval periphery. The finger rest portion 15 accommodates an opening end 16 of a radiation temperature sensor portion, a contact temperature sensor portion 17, and an optical sensor portion 18. There are also an LED 19 and a buzzer for indicating the state of the apparatus, the measuring timing, and so on, by color and by sound, respectively.

Referring to the cross section of the apparatus, the inside of the apparatus will be described. The measurement portion 12 comprises a body surface contact portion 51 on which the body surface is to be placed, a temperature sensor portion 53 for measuring the room temperature and so on, and an optical sensor portion 18. The sensors are covered with a sensor case 54, and the sensors and sensor covers are attached to a substrate 56a. The display portion and the LED are fixed to a substrate 56b. A substrate 56c is fixed to an external case 57. The substrate 56c mounts a microprocessor 55 in addition to the substrates 56a and 56b. The microprocessor 55 includes an operating portion for calculating measurement data, and a control portion for centrally controlling the individual portions.

After turning on the power of the apparatus, the measurement start button 11d is pushed by a finger, for example, whereby a signal is fed from the measurement start button 11d to the microprocessor 55. When a signal is fed to the microprocessor 55 indicating that the body surface such as the finger has come into contact with the measurement portion 12, the microprocessor 55 starts a measurement control routine. Meanwhile, a countdown is displayed on an LCD portion. Alternatively, a signaling LED may be lighted synchronously with the countdown. During the countdown, a measuring LED is lighted. After the countdown is over, the LCD portion indicates the statement "Remove finger from sensor." The apparatus may be arranged such that the power to the apparatus is turned on by pressing the measurement start button 11d for a certain duration of time, such as 3 seconds, so that the measurement start button 11d doubles as the power switch.

When the finger is released from the measurement portion, the apparatus determines the appropriateness of that timing. If the timing is appropriate, the LCD portion displays "Data calculation." If the finger is released before the statement "Remove finger from sensor." the LCD portion displays "Do not release finger before end of countdown." If the finger is released more than 5 seconds later, the LCD portion displays "Release finger immediately after end of countdown." These error messages may be deleted by pressing the measurement start button 11d. If the finger is placed and then released correctly, the apparatus displays the blood sugar level on the LCD portion.

When the power to the apparatus is to be turned off, the power switch is pressed. In the case where the measurement start button 11d doubles as the power switch, the apparatus may be designed such that the power is turned off by pressing the measurement start button 11d for more than 3 seconds. Alternatively, the apparatus may be designed such that the power is turned off by placing the finger on the measurement portion at quick intervals, such as twice within 0.3 to 1 second.

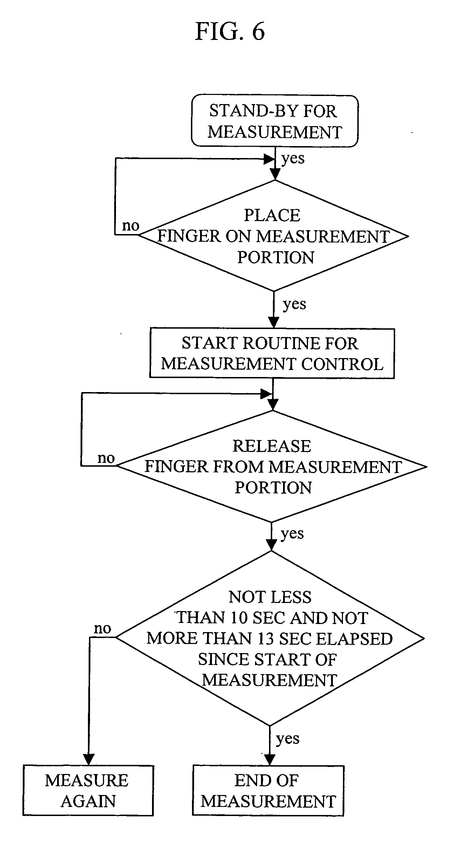

Thus, when the body surface is the finger, the apparatus can be operated from the beginning to the end of measurement with the finger alone. The apparatus may be designed such that the operation including the turning on and off of the power is carried out by the finger alone. Fig. 6 shows the flow of operation when the finger operation is used as a trigger.

Buttons 11a, 11b and 11c are setting buttons for implementing the time setting, history referencing, and so on, which are functions other than that for the measurement of blood sugar level. After the power to the apparatus is turned on, warming up starts. After 30 seconds or more following the warm-up, the apparatus enters an initial state. As any of the buttons 11a, 11b and 11c is pressed at this timing, the apparatus transitions into a function mode not related to measurement. If a predetermined time elapses following the entry into the initial state, the apparatus enters a measurement standby state. Once in the measurement standby state, the apparatus disregards any input from any of the setting buttons 11a, 11b and 11c and thus remains in the standby state. For this purpose, the apparatus includes a button signal processing filter, which is adapted to nullify an input signal from any of the buttons 11a, 11b and 11c when an input from the measurement start button 11d is present.

Once measurement is initiated, the mode of the button signal processing filter is switched, such that all button input signals are invalidated. Thus, none of the buttons is allowed to interrupt a measurement that is being carried out by the apparatus. If the finger is placed on the measurement portion 12 and not more than 10 seconds has elapsed after the start of measurement, the measurement is continued. Namely, the fact that the finger has been placed on the measurement portion 12 for a given time (approximately 10 seconds or less) following the start of measurement is detected by a finger contact recognizing mechanism including detectors for detecting temperature changes in e.g. $T_1$ and $T_3$, thereby obtaining a signal for allowing the measurement to continue. After the measurement is over, the apparatus calculates blood sugar level and displays it on the display.

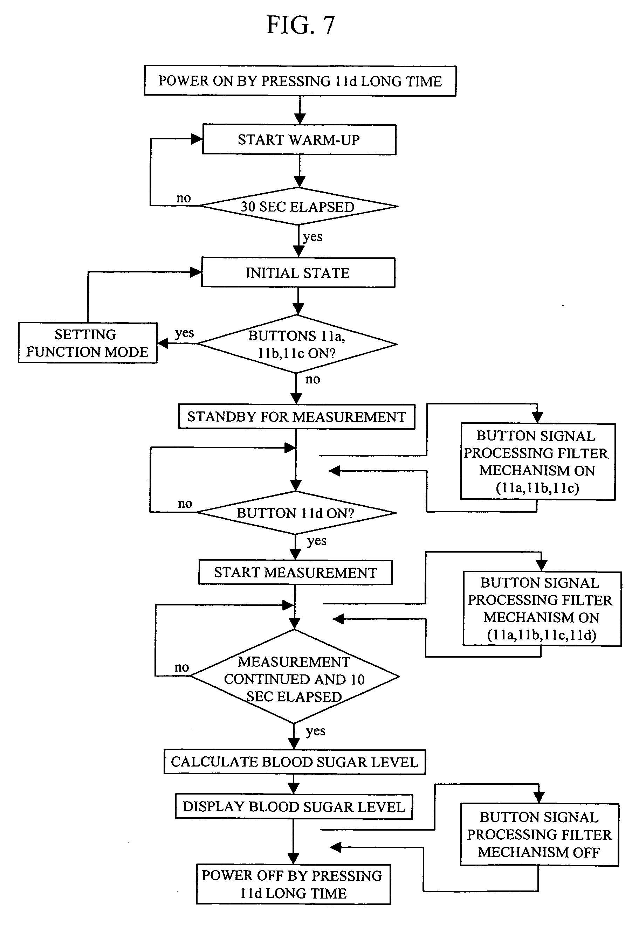

Fig. 7 shows the flow of operation of the apparatus associated with inputs from the buttons. In the following description, it is assumed that the measurement start button 11d doubles as the power switch, and that the other buttons 11a, 11b and 11c are buttons for the setting of the apparatus or for history referencing.

A test subject who is to undergo a blood sugar level measurement presses the button 11d for 3 seconds or longer. This operation causes the apparatus to be turned on. The apparatus then automatically enters the warm-up phase, which lasts for 30 seconds, and then enters the initial state. If any of the buttons 11a, 11b and 11c is pressed at this timing, the apparatus transitions into a setting function mode for the setting of the apparatus. After the setting is complete, the apparatus returns to the initial state, which is then followed by the standby state in preparation for measurement. Once the apparatus is in the standby state, the button signal processing filter mechanism is activated, so that no button input is accepted other than that of the button 11d. Thereafter, as the button 11d is pressed and the finger is placed on the measurement portion, the apparatus detects them and starts measurement. Once measurement starts, any input from any of the buttons 11a, 11b, 11c and 11d is invalidated by the button signal processing filter mechanism. When the measurement is completed and the calculated blood sugar level is displayed on the LCD portion, the button signal processing filter mechanism is deactivated, and the input from the buttons is accepted. Then, if the subject presses the button 11d for 3 seconds or longer, the apparatus is turned off.

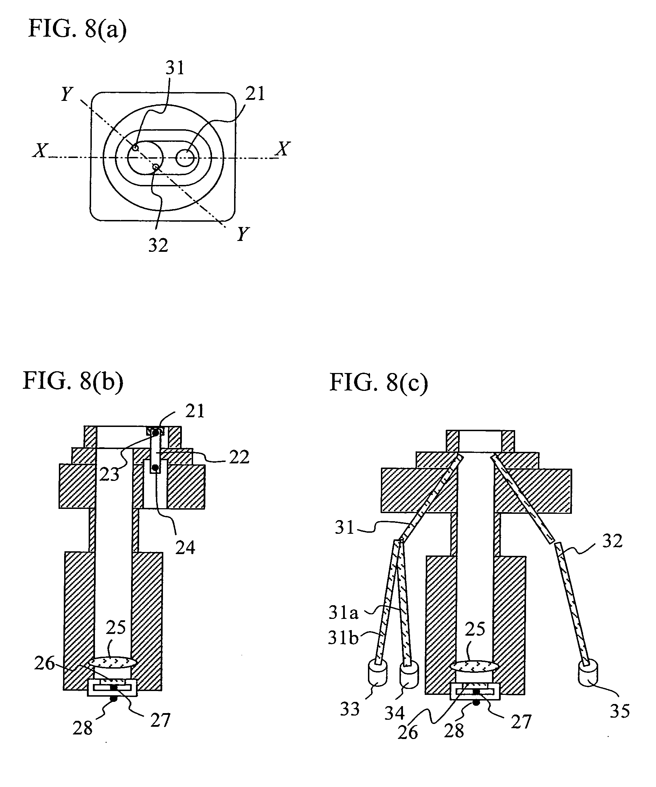

Fig. 8 shows the details of the measurement portion. Fig. 8(a) is a top plan view, (b) is a cross section taken along line XX of (a), and (c) is a cross section taken along YY of (a).

First, temperature measurement by the non-invasive blood sugar level measuring apparatus according to the invention will be described. A thin plate 21 of a highly heat-conductive material, such as gold, is disposed on a portion where a measured portion (ball of the finger) is to come into contact. A bar-shaped heat-conductive member 22 made of a material with a heat conductivity lower than that of the plate 21, such as polyvinylchloride, is thermally connected to the plate 21 and extends into the apparatus. The temperature sensors include a thermistor 23, which is an adjacent temperature detector with respect to the measured portion for measuring the temperature of the plate 21. There is also a thermistor 24, which is an indirect temperature detector with respect to the measured portion for measuring the temperature of a portion of the heat-conducting member away from the plate 21 by a certain distance. An infrared lens 25 is disposed inside the apparatus at such a position that the measured portion (ball of the finger) placed on the finger rest portion 15 can be seen through the lens. Below the infrared lens 25, there is disposed a pyroelectric detector 27 via an infrared radiation-transmitting window 26. Another thermistor 28 is disposed near the pyroelectric detector 27.

Thus, the temperature sensor portion of the measurement portion has four temperature sensors, and they measure four kinds of temperatures as follows:

(1) Temperature on the finger surface (thermistor 23): $T_1$
(2) Temperature of the heat-conducting member (thermistor 24): $T_2$
(3) Temperature of radiation from the finger (pyroelectric detector 27): $T_3$
(4) Room temperature (thermistor 28): $T_4$

If some object approaches over the measurement portion, radiant heat from the object is sensed by the pyroelectric detector 27. When the measured portion, i.e., the finger, comes into contact with the measurement portion, heat from the measured portion is sensed by the thermistor 23. Therefore, the fact that the measured portion is in contact with the measurement portion can be detected when there is an increase in the output of the pyroelectric detector 27 and, at the same time, there is an increase in the temperature measured by the thermistor 23. If the temperature from the thermistor 23 keeps increasing or transitions into a steady state following an increase, it can be known that the measured portion is in contact with the measurement portion continuously. In Fig. 3, the temperature from the pyroelectric detector 27 increases at $t_{start}$. In Fig. 2, the temperature increases at $t_{start}$. Thus, it can be seen that the measured portion came into contact with the measurement portion at $t_{start}$. Alternatively, a pressure sensor or a switch may be provided below the sensor so that the placement of the finger can be ascertained based on a change in a signal from the pressure sensor or on the turning on of the switch.

When the finger is released from the measurement portion, the signal from the pyroelectric detector 27 drops rapidly, and also the temperature from the thermistor 23 starts dropping, such that the release of the finger can be detected. In Fig. 2, because the temperature increases and then enters a rather steady state that continues up to $t_{end}$, it can be concluded that the measured portion left the measurement portion at $t_{end}$. Alternatively, a pressure sensor or a switch may be provided below the sensor so that the departure of the finger can be ascertained based on a change in a signal from the pressure sensor or on the turning off of the switch.

The optical sensor portion 18 will be described. The optical sensor portion measures the hemoglobin concentration and hemoglobin oxygen saturation for obtaining the oxygen supply volume. For measuring the hemoglobin concentration and hemoglobin oxygen saturation, absorbance must be measured at at least two wavelengths. Fig. 8 (c) shows an example of an arrangement for performing the two-wavelength measurement using two light sources 33 and 34 and one detector 35.

Inside the optical sensor portion 18, there are disposed the end portions of two optical fibers 31 and 32. The optical fiber 31 is for irradiating light, and the optical fiber 32 is for receiving light. As shown in Fig. 8(c), the optical fiber 31 is connected to branch fibers 31a and 31b at the ends of which light-emitting diodes 33 and 34 with two different wavelengths are provided. At the end of the optical fiber 32, there is provided a photodiode 35. The light-emitting diode 33 emits light of a wavelength 810 nm. The light-emitting diode 34 emits light of a wavelength 950 nm. The wavelength 810 nm is the iso-absorption wavelength at which the molar absorption coefficients of oxygen-bound hemoglobin and reduced (deoxygenated) hemoglobin are equal. The wavelength 950 nm is the wavelength at which the difference in molar absorption coefficients between the oxygen-bound hemoglobin and the reduced hemoglobin is large.

The two light-emitting diodes 33 and 34 emit light in a time-divided manner, using the timing at which the finger is placed on the finger rest portion as a trigger. The light emitted by the light-emitting diodes 33 and 34 is irradiated via the light-emitting optical fiber 31 onto the finger of the subject. The light with which the finger is irradiated is reflected by the finger skin, incident on the light-receiving optical fiber 32, and then detected by the photodiode 35. When the light with which the finger is irradiated is reflected by the finger skin, some of the light penetrates through the skin and into the tissue, and is then absorbed by the hemoglobin in the blood flowing in capillary blood vessels. The measurement data obtained by the photodiode 35 is reflectance R, and the absorbance is approximated by log (1/R). Irradiation is conducted with light of the wavelengths 810 nm and 950 nm, and R is measured for each, and then log (1/R) is calculated, thereby measuring absorbance $A_1$ for wavelength 810 nm and absorbance $A_2$ for wavelength 950 nm.

When the reduced hemoglobin concentration is [Hb], and the oxygen-bound hemoglobin concentration is [HbO$_2$], absorbance $A_1$ and $A_2$ are expressed by the following equations:

$$A_1 = a \times ([Hb] \times A_{Hb}(810nm) + [HbO_2] \times A_{HbO_2}(810nm))$$

$$= a \times ([Hb] + [HbO_2]) \times A_{HbO_2}(810nm)$$

$$A_2 = a \times ([Hb] \times A_{Hb}(950nm) + [HbO_2] \times A_{HbO_2}(950nm))$$

$$= a \times ([Hb] + [HbO_2]) \times ((1 - \frac{[HbO_2]}{[Hb] + [HbO_2]}) \times A_{Hb}(950nm) + \frac{[HbO_2]}{[Hb] + [HbO_2]} \times A_{HbO_2}(950nm))$$

$A_{Hb}$ (810 nm) and $A_{Hb}$ (950 nm), and $A_{HbO2}$ (810 nm) and $A_{HbO2}$ (950 nm) are molar absorption coefficients of reduced hemoglobin and oxygen-bound hemoglobin, respectively, and are known at the respective wavelengths. Sign a is a proportional coefficient. Based on the above equations, the hemoglobin concentration ($[Hb] + [HbO_2]$) and the hemoglobin oxygen saturation $\{[HbO_2] / ([Hb] + [HbO_2])\}$ can be determined as follows:

$$[Hb] + [HbO_2] = \frac{A_1}{a \times A_{HbO_2}(810nm)}$$

$$\frac{[HbO_2]}{[Hb] + [HbO_2]} = \frac{A_2 \times A_{HbO_2}(810nm) - A_1 \times A_{Hb}(950nm))}{A_1 \times (A_{HbO_2}(950nm) - A_{Hb}(950nm))}$$

While in the above example the hemoglobin concentration and hemoglobin oxygen saturation are measured by measuring absorbance at two wavelengths, it is possible to reduce the influence of interfering components and increase measurement accuracy by measuring at three or more wavelengths.

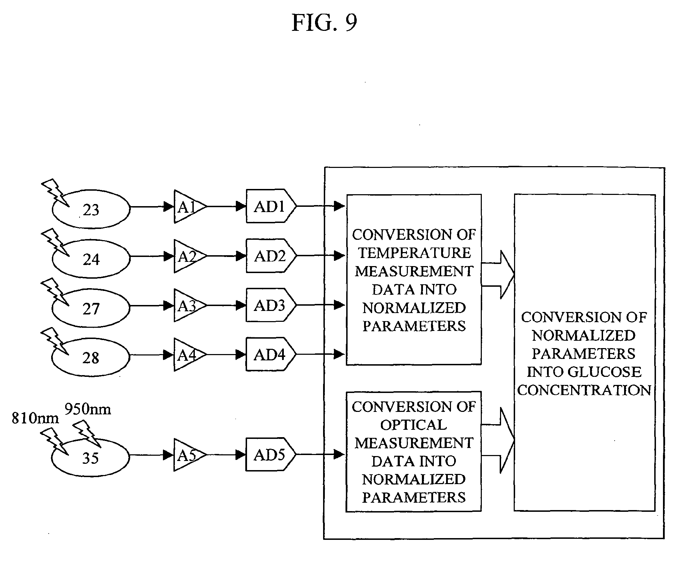

Fig. 9 is a conceptual chart illustrating the flow of data processing in the apparatus. The apparatus according to the present example is equipped with five sensors, namely thermistor 23, thermistor 24, pyroelectric detector 27, thermistor 28 and photodiode 35. The photodiode 35 measures the absorbance at wavelength 810 nm and the absorbance at wavelength 950 nm. Thus, six kinds of measurement values are fed to the apparatus.

Five kinds of analog signals are supplied via amplifiers A1 to A5 and digitally converted by analog/digital converters AD1 to AD5. Based on the digitally converted values, parameters $x_i$ (i=1, 2, 3, 4, 5) are calculated. The following are specific descriptions of $x_i$ (where $e_1$ to $e_5$ are proportionality coefficients):

Parameter proportional to heat radiation

$$x_1 = a_1 \times (T_3)^4$$

Parameter proportional to heat convection

$$x_2 = a_2 \times (T_4 - T_3)$$

Parameter proportional to hemoglobin concentration

$$x_3 = e_3 \times \left( \frac{A_1}{a \times A_{HbO_2}(810nm)} \right)$$

Parameter proportional to hemoglobin saturation

$$x_4 = e_4 \times \left( \frac{A_2 \times A_{HbO_2}(810nm) - A_1 \times A_{Hb}(950nm))}{A_1 \times (A_{HbO_2}(950nm) - A_{Hb}(950nm))} \right)$$

Parameter proportional to blood flow volume

$$x_5 = e_5 \left( \frac{1}{t_{CONT} \times (S_1 - S_2)} \right)$$

Then, normalized parameters are calculated from mean values and standard deviations of parameters $x_i$ obtained from actual data from large numbers of able-bodied people and diabetic patients. A normalized parameter $X_i$ (where i=1, 2, 3, 4, 5) is calculated from each parameter $x_i$ according to the following equation:

$$X_i = \frac{x_i - \bar{x}_i}{SD(x_i)}$$

where

$x_i$: parameter
$\bar{x}_i$ : mean value of the parameter
$SD(x_i)$: standard deviation of the parameter

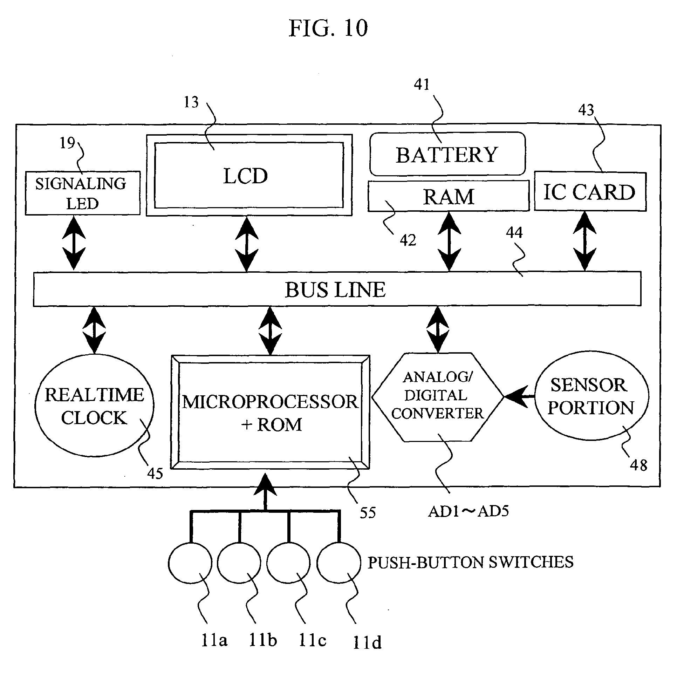

Calculations are conducted to convert the above five normalized parameters into a glucose concentration to be eventually displayed. Fig. 10 schematically shows an example of the inside of the apparatus. The LCD portion 13 and the signaling LED 19 are disposed at positions within the field of view of the user. The push buttons 11a to 11d are connected to the microprocessor 55. The microprocessor 55 includes a ROM for storing software. External instructions can be entered into the microprocessor 55 by pressing the buttons 11a to 11d.

Programs necessary for computations are stored in the ROM built inside a ROM in the apparatus. Memory areas necessary for computations are ensured in a RAM 42 built inside the apparatus. The analog signals from the sensor portion are converted into digital signals by analog/digital converters AD1 to AD5, transferred via a bus line 44, and are then subjected to calculation processes in the microprocessor using the functions stored in the ROM. Depending on the result of the calculation processes, the signaling LED 19 emits light or blinks. When the signal from the sensor portion indicates that the finger is placed, the LCD portion displays countdown in response to an instruction from a real-time clock 45, while a blood sugar measuring program stored in the ROM is started. The result of the calculation processes may be stored in an IC card 43 as well as being displayed on the LCD portion. When a battery 41 runs low, the LCD portion may display a warning, or the signaling LED may be caused to emit light or blink.

The ROM stores, as a constituent element of the program necessary for the computations, a function for determining glucose concentration C in particular. The function is defined as follows. C is expressed by a below-indicated equation (1), where $a_i$ (i=0, 1, 2, 3, 4, 5) is determined from a plurality of pieces of measurement data in advance according to the following procedure:

(1) A multiple regression equation is created that indicates the relationship between the normalized parameter and the glucose concentration C.
(2) Normalized equations (simultaneous equations) relating to the normalized parameter are obtained from an equation obtained by the least-squares method.
(3) Values of coefficient a; (i=0, 1, 2, 3, 4, 5) are determined from the normalized equation and then substituted into the multiple regression equation.

Initially, the regression equation (1) indicating the relationship between the glucose concentration C and the normalized parameters $X_1$, $X_2$, $X_3$, $X_4$ and $X_5$ is formulated.

$$C = f(X_1, X_2, X_3, X_4, X_5)$$
$$= a_0 + a_1 X_1 + a_2 X_2 + a_3 X_3 + a_4 X_4 + a_5 X_5 \quad ......(1)$$

Then, the least-squares method is employed to obtain a multiple regression equation that would minimize the error with respect to a measured value $C_i$ of glucose concentration according to an enzyme electrode method. When the sum of squares of the residual is D, D is expressed by the following equation (2):

$$D = \sum_{i=1}^{n} d_i^2$$

$$= \sum_{i=1}^{n} (C_i - f(X_{i1}, X_{i2}, X_{i3}, X_{i4}, X_{i5}))^2$$

$$= \sum_{i=1}^{n} \{C_i - (a_0 + a_1 X_{i1} + a_2 X_{i2} + a_3 X_{i3} + a_4 X_{i4} + a_5 X_{i5})\}^2 \quad ......(2)$$

The sum of squares of the residual D becomes minimum when partial differentiation of equation (2) with respect to $a_0$, $a_2$, ..., $a_5$ gives zero. Thus, we have the following equations:

$$\frac{\partial D}{\partial a_0} = -2\sum_{i=1}^{n} \{C_i - (a_0 + a_1 X_{i1} + a_2 X_{i2} + a_3 X_{i3} + a_4 X_{i4} + a_5 X_{i5})\} = 0$$

$$\frac{\partial D}{\partial a_1} = -2\sum_{i=1}^{n} X_{i1}\{C_i - (a_0 + a_1 X_{i1} + a_2 X_{i2} + a_3 X_{i3} + a_4 X_{i4} + a_5 X_{i5})\} = 0$$

$$\frac{\partial D}{\partial a_2} = -2\sum_{i=1}^{n} X_{i2}\{C_i - (a_0 + a_1 X_{i1} + a_2 X_{i2} + a_3 X_{i3} + a_4 X_{i4} + a_5 X_{i5})\} = 0$$

$$\frac{\partial D}{\partial a_3} = -2\sum_{i=1}^{n} X_{i3}\{C_i - (a_0 + a_1 X_{i1} + a_2 X_{i2} + a_3 X_{i3} + a_4 X_{i4} + a_5 X_{i5})\} = 0$$

$$\frac{\partial D}{\partial a_4} = -2\sum_{i=1}^{n} X_{i4}\{C_i - (a_0 + a_1 X_{i1} + a_2 X_{i2} + a_3 X_{i3} + a_4 X_{i4} + a_5 X_{i5})\} = 0$$

$$\frac{\partial D}{\partial a_5} = -2\sum_{i=1}^{n} X_{i5}\{C_i - (a_0 + a_1 X_{i1} + a_2 X_{i2} + a_3 X_{i3} + a_4 X_{i4} + a_5 X_{i5})\} = 0 \quad ......(3)$$

When the mean values of C and $X_1$ to $X_5$ are $C_{mean}$ and $X_{1mean}$ to $X_{5mean}$, respectively, since $X_{imean}=0$ (i=1 to 5), equation (1) yields equation (4) thus:

$$a_0 = C_{mean} - a_1 X_{1mean} - a_2 X_{2mean} - a_3 X_{3mean} - a_4 X_{4mean} - a_5 X_{5mean}$$
$$= C_{mean} \qquad\qquad ......(4)$$

The variation and covariation between the normalized parameters are expressed by equation (5). Covariation between the normalized parameter $X_i$ (i=1 to 5) and C is expressed by equation (6).

$$S_{ij} = \sum_{k=1}^{n}(X_{ki} - X_{imean})(X_{kj} - X_{jmean}) = \sum_{k=1}^{n} X_{ki} X_{kj} \quad (i,j = 1,2,..5) \quad ......(5)$$

$$S_{iC} = \sum_{k=1}^{n}(X_{ki} - X_{imean})(C_k - C_{mean}) = \sum_{k=1}^{n} X_{ki}(C_k - C_{mean}) \quad (i = 1,2,..5) \quad ......(6)$$

Substituting equations (4), (5), and (6) into equation (3) and rearranging yields simultaneous equations (normalized equations) (7). Solving equations (7) yields $a_1$ to $a_5$.

$$a_1 S_{11} + a_2 S_{12} + a_3 S_{13} + a_4 S_{14} + a_5 S_{15} = S_{1C}$$

$$a_1 S_{21} + a_2 S_{22} + a_3 S_{23} + a_4 S_{24} + a_5 S_{25} = S_{2C}$$

$$a_1 S_{31} + a_2 S_{32} + a_3 S_{33} + a_4 S_{34} + a_5 S_{35} = S_{3C}$$

$$a_1 S_{41} + a_2 S_{42} + a_3 S_{43} + a_4 S_{44} + a_5 S_{45} = S_{4C}$$

$$a_1 S_{51} + a_2 S_{52} + a_3 S_{53} + a_4 S_{54} + a_5 S_{55} = S_{5C} \qquad\qquad (7)$$

Constant term $a_0$ is obtained by means of equation (4). The thus obtained $a_i$ (i=0, 1, 2, 3, 4, 5) is stored in ROM at the time of manufacture of the apparatus. In actual measurement using the apparatus, the normalized parameters $X_1$ to $X_5$ obtained from the measured values are substituted into regression equation (1) to calculate the glucose concentration C.

Hereafter, an example of the process of calculating the glucose concentration will be described. The coefficients in equation (1) are determined in advance based on a large quantity of data obtained from able-bodied persons and diabetic patients. The ROM in the microprocessor stores the following formula for the calculation of glucose concentration:

$$C = 99.4 + 18.3 \times X_1 - 20.2 \times X_2 - 23.7 \times X_3 - 22.0 \times X_4 - 25.9 \times X_5$$

$X_1$ to $X_5$ are the results of normalization of parameters $x_1$ to $x_5$. Assuming the distribution of the parameters is normal, 95% of the normalized parameters take on values between -2 and +2.

In an example of measured values for an able-bodied person, substituting normalized parameters $X_1$=-0.06, $X_2$=+0.04 and $X_3$=+0.05, $X_4$=-0.12 and $X_5$=+0.10 in the above equation yields C=96 mg/dL. In an example of measured values for a diabetic patient, substituting normalized parameters $X_1$=+1.15, $X_2$=-1.02, $X_3$=-0.83, $X_4$=-0.91 and $X_5$=-1.24 in the equation yields C=213 mg/dL.

Hereafter, the results of measurement by the conventional enzymatic electrode method and those by the embodiment of the invention will be described. In the enzymatic electrode method, a blood sample is reacted with a reagent and the amount of resultant electrons is measured to determine blood sugar level. When the glucose concentration was 89 mg/dL according to the enzymatic electrode method in an example of measured values for an able-bodied person, substituting normalized parameters $X_1$=-0.06, $X_2$=+0.04, $X_3$=+0.05, $X_4$=-0.12 and $X_5$=+0.10 obtained by measurement at the same time according to the inventive method into the above equation yield C=96 mg/dL. Further, when the glucose concentration was 238 mg/dL according to the enzymatic electrode method in an example of measurement values for a diabetic patient, substituting $X_1$=+1.15, $X_2$=-1.02, $X_3$=-0.83, $X_4$=-0.91 and $X_5$=-1.24 obtained by measurement at the same time according to the inventive method yields C=213 mg/dL.

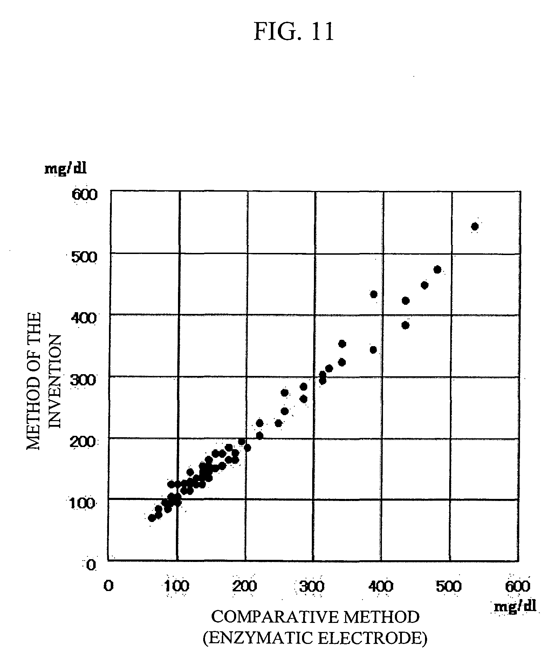

Fig. 11 shows a chart plotting on the vertical axis the values of glucose concentration calculated by the inventive method and on the horizontal axis the values of glucose concentration measured by the enzymatic electrode method, based on measurement values obtained from a plurality of patients. A good correlation is obtained by measuring the oxygen supply volume and blood flow volume according to the invention (correlation coefficient = 0.9324).

In the above-described embodiment, the parameters relating to blood hemoglobin concentration and blood hemoglobin oxygen saturation are obtained by spectroscopically measuring the hemoglobin in blood. However, the hemoglobin concentration is stable in persons without such symptoms as anemia, bleeding or erythrocytosis. The hemoglobin concentration is normally in the range between 13 to 18 g/dL for males and between 12 to 17 g/dL for females, and the range of variation of hemoglobin concentration from the normal values is 5 to 6%. Further, the weight of the term in the aforementioned formula for calculating blood sugar level is smaller than other terms. Therefore, the hemoglobin concentration can be treated as a constant without greatly lowering the measurement accuracy. Similarly, the hemoglobin oxygen saturation is stable between 97 to 98% if the person is undergoing aerial respiration at atmospheric pressure, at rest and in a relaxed state. Thus the hemoglobin concentration and the hemoglobin oxygen saturation can be treated as constants, and the oxygen supply volume can be determined from the product of the hemoglobin concentration constant, the hemoglobin oxygen saturation constant and the blood flow volume.

By treating the hemoglobin concentration and hemoglobin oxygen saturation as constants, the sensor arrangement for measuring blood sugar level can be simplified by removing the optical sensors, for example. Further, by eliminating the time necessary for optical measurement and the processing thereof, the procedure for blood sugar level measurement can be accomplished in less time.

Because the hemoglobin oxygen saturation takes on a stable value when at rest, in particular, by treating the hemoglobin concentration and hemoglobin oxygen saturation as constants, the measurement accuracy for blood sugar level measurement when at rest can be increased, and the procedure blood sugar level measurement can be accomplished in less time. By "when at rest" herein is meant the state in which the test subject has been either sitting on a chair or lying and thus moving little for approximately five minutes.

Hereafter, an embodiment will be described in which the blood hemoglobin concentration and blood hemoglobin oxygen saturation are treated as constants. This embodiment is similar to the above-described embodiment except that the blood hemoglobin concentration and blood hemoglobin oxygen saturation are treated as constants, and therefore the following description mainly concerns the differences from the earlier embodiment.

In the present embodiment, the hemoglobin concentration and hemoglobin oxygen saturation shown in Fig. 4 are not measured but treated as constants. Therefore, the measurement portion of the present embodiment has the structure of the measurement portion of the earlier embodiment shown in Fig. 8 from which the light sources 33 and 34, photodiode 35 and optical fibers 31 and 32 are removed. Parameters used in the present embodiment are parameter $x_1$ proportional to heat radiation, parameter $x_2$ related to heat convection, and parameter $x_3$ proportional to the oxygen supply volume (hereafter, parameter proportional to oxygen supply volume will be indicated as $x_3$). From these parameters, normalized parameters are calculated in the manner described above, and a glucose concentration is calculated based on the three normalized parameters $X_i$ (i=1, 2, 3). During data processing, the "CONVERSION OF OPTICAL MEASUREMENT DATA INTO NORMALIZED PARAMETERS" (see Fig. 9), which is necessary in the previous embodiment, can be omitted.

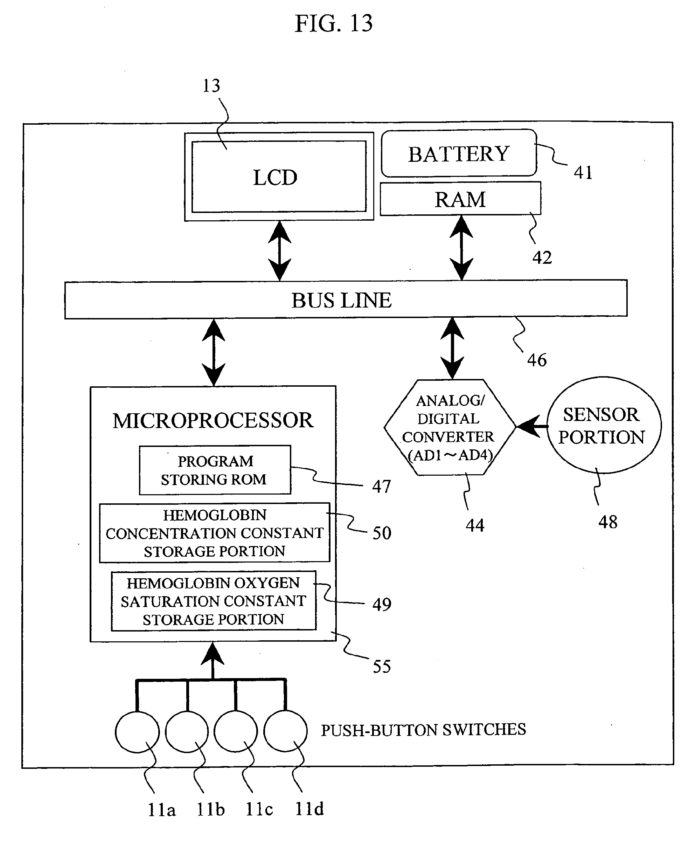

Fig. 13 shows a functional block diagram of the apparatus according to the embodiment. The apparatus runs on battery 41. A signal measured by sensor portion 48 including a temperature sensor is fed to analog/digital converters 44 (AD1 to AD4) provided for individual signals and is converted into a digital signal. Analog/digital converters AD1 to AD4, LCD 13 and RAM 42 are peripheral circuits for microprocessor 55. They are accessed by the microprocessor 55 via bus line 46. The push buttons 11 a to 11d are connected to microprocessor 55. The microprocessor 55 includes the ROM for storing software. By pressing the buttons 11a to 11d, external instructions can be entered into microprocessor 55.

The ROM 47 included in the microprocessor 55 stores a program necessary for computations, i.e., it has the function of an arithmetic unit. The microprocessor 55 further includes a hemoglobin concentration constant storage portion 50 for storing hemoglobin concentration constants, and a hemoglobin oxygen saturation constant storage portion 49 for storing hemoglobin oxygen saturation constants. After the measurement of the finger is finished, the computing program calls optimum constants from the hemoglobin concentration storage portion 50 and hemoglobin oxygen saturation constant storage portion 49 and perform calculations. A memory area necessary for computations is ensured in the RAM 42 similarly incorporated into the apparatus. The result of computations is displayed on the LCD portion.

The ROM stores, as a constituent element of the program necessary for the computations, a function for determining glucose concentration C in particular. The function is defined as follows. C is expressed by a below-indicated equation (8), where $a_i$ (i=0, 1, 2, 3) is determined from a plurality of pieces of measurement data in advance according to the following procedure:

(1) A multiple regression equation is created that indicates the relationship between the normalized parameter and the glucose concentration C.
(2) Normalized equations (simultaneous equations) relating to the normalized parameter are obtained from an equation obtained by the least-squares method.
(3) Values of coefficient $a_i$ (i=0, 1, 2, 3) are determined from the normalized equation and then substituted into the multiple regression equation.

Initially, the regression equation (8) indicating the relationship between the glucose concentration C and the normalized parameters $X_1$, $X_2$ and $X_3$ is formulated.

$$C = f(X_1, X_2, X_3)$$
$$= a_0 + a_1 X_1 + a_2 X_2 + a_3 X_3 \quad ......(8)$$

Then, the least-squares method is employed to obtain a multiple regression equation that would minimize the error with respect to a measured value $C_i$ of glucose concentration according to an enzyme electrode method. When the sum of squares of the residual is D, D is expressed by the following equation (9):

$$D = \sum_{i=1}^{n} d_i^2$$
$$= \sum_{i=1}^{n} (C_i - f(X_{i1}, X_{i2}, X_{i3}))^2$$
$$= \sum_{i=1}^{n} \{C_i - (a_0 + a_1 X_{i1} + a_2 X_{i2} + a_3 X_{i3})\}^2 \quad ......(9)$$

The sum of squares of the residual D becomes minimum when partial differentiation of equation (9) with respect to $a_0$ to $a_3$ gives zero. Thus, we have the following equations:

$$\frac{\partial D}{\partial a_0} = -2\sum_{i=1}^{n} \{C_i - (a_0 + a_1 X_{i1} + a_2 X_{i2} + a_3 X_{i3})\} = 0$$

$$\frac{\partial D}{\partial a_1} = -2\sum_{i=1}^{n} X_{i1}\{C_i - (a_0 + a_1 X_{i1} + a_2 X_{i2} + a_3 X_{i3})\} = 0$$

$$\frac{\partial D}{\partial a_2} = -2\sum_{i=1}^{n} X_{i2}\{C_i - (a_0 + a_1 X_{i1} + a_2 X_{i2} + a_3 X_{i3})\} = 0$$

$$\frac{\partial D}{\partial a_3} = -2\sum_{i=1}^{n} X_{i3}\{C_i - (a_0 + a_1 X_{i1} + a_2 X_{i2} + a_3 X_{i3})\} = 0 \quad ......(10)$$

When the mean values of C and $X_1$ to $X_3$ are $C_{mean}$ and $X_{1mean}$ to $X_{3mean}$, respectively, since $X_{imean}=0$ (i=1 to 3), equation (8) yields equation (11) thus:

$$a_0 = C_{mean} - a_1 X_{1mean} - a_2 X_{2mean} - a_3 X_{3mean}$$
$$= C_{mean} \qquad\qquad ......(11)$$

The variation and covariation between the normalized parameters are expressed by equation (12). Covariation between the normalized parameter $X_i$ (i=1 to 3) and C is expressed by equation (13).

$$S_{ij} = \sum_{k=1}^{n}(X_{ki} - X_{imean})(X_{kj} - X_{jmean}) = \sum_{k=1}^{n} X_{ki}X_{kj} \quad (i,j = 1,2,3) \quad ......(12)$$

$$S_{iC} = \sum_{k=1}^{n}(X_{ki} - X_{imean})(C_k - C_{mean}) = \sum_{k=1}^{n} X_{ki}(C_k - C_{mean}) \quad (i = 1,2,3) \quad ......(13)$$

Substituting equations (11), (12), and (13) into equation (10) and rearranging yields simultaneous equations (normalized equations) (14). Solving equations (14) yields $a_1$ to $a_3$.

$$a_1 S_{11} + a_2 S_{12} + a_3 S_{13} = S_{1C}$$

$$a_1 S_{21} + a_2 S_{22} + a_3 S_{23} = S_{2C}$$

$$a_1 S_{31} + a_2 S_{32} + a_3 S_{33} = S_{3C} \qquad\qquad (14)$$

Constant term $a_0$ is obtained by means of equation (11). The thus obtained $a_i$ (i=0, 1, 2, 3) is stored in ROM at the time of manufacture of the apparatus. In actual measurement using the apparatus, the normalized parameters $X_1$ to $X_3$ obtained from the measured values are substituted into regression equation (8) to calculate the glucose concentration C.

Hereafter, an example of the process of calculating the glucose concentration will be described. The coefficients in equation (8) are determined in advance based on a large quantity of data obtained from able-bodied persons and diabetic patients. The ROM in the microprocessor stores the following formula for the calculation of glucose concentration:

$$C = 101.7 + 25.8 \times X_1 - 23.2 \times X_2 - 12.9 \times X_3$$

$X_1$ to $X_3$ are the results of normalization of parameters $x_1$ to $x_3$. Assuming the distribution of the parameters is normal, 95% of the normalized parameters take on values between -2 and +2.

In an example of measured values for an able-bodied person, substituting normalized parameters $X_1$=-0.06, $X_2$=+0.04 and $X_3$=+0.10 in the above equation yields C=101 mg/dL. In an example of measured values for a diabetic patient, substituting normalized parameters $X_1$=+1.35, $X_2$=-1.22 and $X_3$=-1.24 in the equation yields C=181 mg/dL. In the above equation, the hemoglobin concentration and hemoglobin oxygen saturation are rendered into constants of 15 g/dL and 97%, respectively.

Hereafter, the results of measurement by the conventional enzymatic electrode method and those by the embodiment of the invention will be described. In the enzymatic electrode method, a blood sample is reacted with a reagent and the amount of resultant electrons is measured to determine glucose concentration. When the glucose concentration was 93 mg/dL according to the enzymatic electrode method in an example of measured values for an able-bodied person, substituting normalized parameters $X_1$=-0.06, $X_2$=+0.04 and $X_3$=+0.10 obtained by measurement at the same time according to the inventive method into the above equation yielded C=101 mg/dL. Further, when the glucose concentration was 208 mg/dL according to the enzymatic electrode method in an example of measurement values for a diabetic patient, substituting $X_1$=+1.35, $X_2$=-1.22 and $X_3$=-1.24 obtained by measurement at the same time according to the inventive method yielded C=181 mg/dL. Although the calculation results indicate an error of about 13%, this level of accuracy is considered sufficient because normally errors between 15% and 20% are considered acceptable in blood sugar level measuring apparatuses in general. Thus, it has been confirmed that the method of the invention can allow glucose concentrations to be determined with high accuracy.

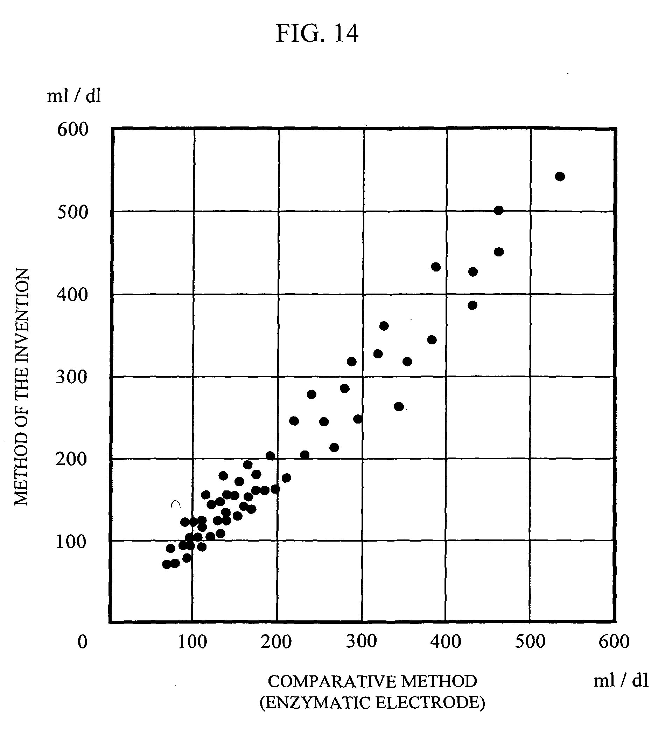

Fig. 14 shows a chart plotting on the vertical axis the values of glucose concentration calculated by the inventive method and on the horizontal axis the values of glucose concentration measured by the enzymatic electrode method, based on measurement values obtained from a plurality of patients. A good correlation is obtained by measuring according to the invention (correlation coefficient = 0.8932).

**Claims**

1.  A blood sugar level measuring apparatus comprising:

    a heat amount measurement portion for measuring a plurality of temperatures derived from a body surface and obtaining information used for calculating the amount of heat transferred by convection and the amount of heat transferred by radiation, both related to the dissipation of heat from said body surface;
    an oxygen level measuring portion for obtaining information about blood oxygen level;
    a storage portion for storing a relationship between parameters corresponding to said plurality of temperatures and blood oxygen level and blood sugar levels;
    a calculating portion which converts a plurality of measurement values fed from said heat amount measuring portion and said oxygen level measurement portion into said parameters, and computes a blood sugar level by applying said parameters to said relationship stored in said storage portion;
    a display portion for displaying the blood sugar level calculated by said calculating portion;
    a plurality of operation buttons including a measurement start button for instructing the start of a measurement, and control buttons for performing controls other than the instruction for starting a measurement; and
    a button signal processing filter mechanism for processing an input signal from said operation buttons, wherein:

    said oxygen level measurement portion includes a blood flow volume measurement portion for obtaining information about blood flow volume, and an optical measurement portion for obtaining blood hemoglobin concentration and hemoglobin oxygen saturation, wherein said blood flow volume measurement portion includes:

    a body-surface contact portion;
    an adjacent temperature detector disposed adjacent to said body-surface contact portion;
    an indirect temperature detector for detecting the concentration at a position spaced apart from said body-surface contact portion; and
    a heat conducting member connecting said body-surface contact portion and said indirect temperature detector.

2.  The blood sugar level measuring apparatus according to claim 1, wherein said button signal processing filter mechanism is adapted to invalidate an input signal from the operation buttons other than said measurement start button when the apparatus is in a measurement standby state.

3.  The blood sugar level measuring apparatus according to claim 1, wherein said button signal processing filter mechanism is adapted to invalidate an input signal from said plurality of operation buttons during measurement.

4.  The blood sugar level measuring apparatus according to claim 1, wherein said measurement start button also serves as a power switch of the apparatus.

5.  A blood sugar level measuring apparatus comprising:

    an ambient temperature measuring device for measuring ambient temperature;
    a body-surface contact portion to which a body surface is brought into contact;
    a radiant heat detector for measuring radiant heat from said body surface;

a heat conducting member disposed in contact with said body-surface contact portion;

an adjacent temperature detector disposed adjacent to said body-surface contact portion;

an indirect temperature detector disposed at a position that is adjacent to said heat conducting member and that is spaced apart from said body-surface contact portion, said indirect temperature detector measuring temperature at the position spaced apart from said body-surface contact portion;

a light source for irradiating said body-surface contact portion light with at least two different wavelengths;

a light detector for detecting reflected light produced as said light is reflected by said body surface;

a converter for converting outputs from said adjacent temperature detector, said indirect temperature detector, said ambient temperature detector, said radiant temperature detector and said light detector, into parameters;

a calculating portion in which a relationship between said parameters and blood sugar levels is stored in advance, and which calculates a blood sugar level by applying said parameters to said relationship;

a display for displaying the blood sugar level outputted from said calculating portion;

a plurality of operation buttons including a measurement start button for instructing the start of a measurement, and control buttons for performing controls other than the instruction for starting a measurement; and

a button signal processing filter mechanism for processing an input signal from operation buttons.

**6.** The blood sugar level measuring apparatus according to claim 5, wherein said button signal processing filter mechanism is adapted to invalidate an input signal from said operation buttons other than said measurement start button when the apparatus is in a measurement standby state.

**7.** The blood sugar level measuring apparatus according to claim 5, wherein said button signal processing filter mechanism is adapted to invalidate an input signal from said plurality of operation buttons during measurement.

**8.** The blood sugar level measuring apparatus according to claim 5, wherein said measurement start button also serves as a power button of the apparatus.

**9.** A blood sugar level measuring apparatus comprising:

an ambient temperature measuring device for measuring ambient temperature;

a body-surface contact portion to which a body surface is brought into contact;

a radiant heat detector for measuring radiant heat from said body surface;

a heat conducting member disposed in contact with said body-surface contact portion;

an adjacent temperature detector disposed adjacent to said body-surface contact portion;

an indirect temperature detector disposed at a position that is adjacent to said heat conducting member and that is spaced apart from said body-surface contact portion, said indirect temperature detector measuring temperature at the position spaced apart from said body-surface contact portion;

a storage portion where information about blood hemoglobin concentration and blood hemoglobin oxygen saturation is stored;

a converter for converting outputs from said adjacent temperature detector, said indirect temperature detector, said ambient temperature measuring device and said radiant heat detector, into a plurality of parameters;

a calculating portion in which a relationship between said parameters and blood sugar levels is stored, said calculating portion including a processing portion for calculating a blood sugar level by applying said parameters to said relationship;

a display for displaying the blood sugar level outputted from said calculating portion;

a plurality of operation buttons including a measurement start button for instructing the start of a measurement, and control buttons for performing controls other than the instruction for starting a measurement; and

a button signal processing filter mechanism for processing an input signal from said operation buttons.

**10.** The blood sugar level measuring apparatus according to claim 9, wherein said button signal processing filter mechanism is adapted to invalidate an input signal from said operation buttons other than said measurement start button when the apparatus is in a measurement standby state.

**11.** The blood sugar level measuring apparatus according to claim 9, wherein said button signal processing filter mechanism is adapted to invalidate an input signal from said plurality of operation buttons during measurement.

**12.** The blood sugar level measuring apparatus according to claim 9, wherein said measurement start button also serves as a power button of the apparatus.

FIG. 1

## FIG. 2

## FIG. 3

# FIG. 4

CONTACT
TEMPERATURE
$T_1, T_2$

RADIATION
TEMPERATURE
$T_3$

ROOM
TEMPERATURE
$T_4$

HEMOGLOBIN
ABSORBANCE
$A_1$

HEMOGLOBIN
ABSORBANCE
$A_2$

AMOUNT OF HEAT
TRANSFER BY CONVECTION

AMOUNT OF HEAT
TRANSFER BY RADIATION

HEAT DISSIPATION
AMOUNT

BLOOD FLOW VOLUME

HEMOGLOBIN
CONCENTRATION

HEMOGLOBIN OXYGEN
SATURATION

OXYGEN SUPPLY
VOLUME

20

FIG. 5

FIG. 6

```
        ┌─────────────────────┐
        │  STAND-BY FOR       │
        │  MEASUREMENT        │
        └─────────────────────┘
                   │
                   ▼ yes
              ╱─────────╲
             ╱   PLACE    ╲
      no ╱ FINGER ON MEASUREMENT ╲
         ╲      PORTION      ╱
          ╲───────────────╱
                   │
                   ▼ yes
        ┌─────────────────────┐
        │  START ROUTINE FOR  │
        │ MEASUREMENT CONTROL │
        └─────────────────────┘
                   │
                   ▼
              ╱─────────╲
             ╱  RELEASE   ╲
   no ╱ FINGER FROM MEASUREMENT ╲
      ╲       PORTION      ╱
       ╲───────────────╱
                   │
                   ▼ yes
              ╱─────────╲
             ╱ NOT LESS   ╲
            ╱ THAN 10 SEC AND NOT ╲
   no ╱ MORE THAN 13 SEC ELAPSED ╲
      ╲    SINCE START OF    ╱
       ╲   MEASUREMENT   ╱
        ╲─────────────╱
         │                │
         ▼                ▼ yes
 ┌───────────┐    ┌───────────┐
 │ MEASURE   │    │  END OF   │
 │  AGAIN    │    │MEASUREMENT│
 └───────────┘    └───────────┘
```

## FIG. 7

```
┌─────────────────────────────────────┐
│ POWER ON BY PRESSING 11d LONG TIME   │
└─────────────────────────────────────┘
                  │
                  ▼
        ┌────────────────────┐
  ┌────▶│   START WARM-UP     │
  │     └────────────────────┘
  │                │
  │                ▼
  │  no    ◇───────────────◇
  └────────  30 SEC ELAPSED
          ◇───────────────◇
                  │ yes
                  ▼
        ┌────────────────────┐
  ┌────▶│   INITIAL STATE     │
  │     └────────────────────┘
  │                │
  │                ▼
┌──────────┐ yes  ◇───────────────◇
│ SETTING  │◀──────  BUTTONS 11a,
│ FUNCTION │       11b,11c ON?
│  MODE    │       ◇───────────────◇
└──────────┘              │ no
                          ▼
        ┌────────────────────────────┐      ┌──────────────────────┐
        │   STANDBY FOR MEASUREMENT   │      │   BUTTON SIGNAL       │
        └────────────────────────────┘      │ PROCESSING FILTER     │
                  │                    ◀─────│ MECHANISM ON          │
  ┌──────────────▶│                          │ (11a,11b,11c)         │
  │               ▼                          └──────────────────────┘
  │  no    ◇───────────────◇
  └────────  BUTTON 11d ON?
          ◇───────────────◇
                  │ yes
                  ▼
        ┌────────────────────┐             ┌──────────────────────┐
        │  START MEASUREMENT  │             │   BUTTON SIGNAL       │
        └────────────────────┘             │ PROCESSING FILTER     │
                  │                    ◀────│ MECHANISM ON          │
  ┌──────────────▶│                         │ (11a,11b,11c,11d)     │
  │               ▼                         └──────────────────────┘
  │        ◇───────────────◇
  │  no    MEASUREMENT
  └────────CONTINUED AND 10
          SEC ELAPSED
          ◇───────────────◇
                  │ yes
                  ▼
        ┌────────────────────────────┐
        │  CALCULATE BLOOD SUGAR LEVEL │
        └────────────────────────────┘
                  │
                  ▼
        ┌────────────────────────────┐      ┌──────────────────────┐
        │  DISPLAY BLOOD SUGAR LEVEL  │      │   BUTTON SIGNAL       │
        └────────────────────────────┘      │ PROCESSING FILTER     │
                  │                    ◀─────│ MECHANISM OFF         │
                  ▼                          └──────────────────────┘
        ┌────────────────────────────┐
        │ POWER OFF BY PRESSING        │
        │ 11d LONG TIME                │
        └────────────────────────────┘
```

FIG. 8(a)

FIG. 8(b)

FIG. 8(c)

FIG. 9

EP 1 568 309 A1

FIG. 10

26

FIG. 11

FIG. 12(a)

FIG. 12(b)

## FIG. 13

FIG. 14

**European Patent Office**

## EUROPEAN SEARCH REPORT

Application Number

EP 04 00 7593

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | WO 01/28414 A (KAUFMANN-KIM, YUN-OAK; CHO, OK-KYUNG) 26 April 2001 (2001-04-26) | 1,5 | A61B5/00 G01K17/00 |
| Y | * page 1, lines 6-15 * <br> * page 3, line 19 - page 5, line 2 * <br> * page 8, line 30 - page 9, line 24 * <br> * page 11, lines 1-9 * <br> * page 12, line 28 - page 13, line 25 * <br> * page 15, line 26 - page 17, line 2 * <br> ----- | 2-4,6-8 | |
| X | WO 96/01075 A (MED SCIENCE GMBH; CHO, OK-KYUNG; HOLZGREVE, BIRGIT) 18 January 1996 (1996-01-18) | 9 | |
| Y | * page 12, line 14 - page 13, line 26 * <br> * page 14, line 15 - page 15, line 12 * <br> ----- | 10-12 | |
| E | EP 1 484 006 A (HITACHI, LTD) 8 December 2004 (2004-12-08) <br> * paragraphs [0038], [0039]; figure 5 * <br> ----- | 1,5,9 | |
| Y | US 6 270 455 B1 (BROWN STEPHEN J) 7 August 2001 (2001-08-07) <br> * column 13, lines 7-14 * <br> * column 15, lines 13-23 * <br> * column 24, line 33 - column 25, line 52 * <br> ----- | 2,6,10 | TECHNICAL FIELDS SEARCHED (Int.Cl.7) <br><br> A61B G01K |
| Y | EP 0 387 630 A (MILES INC) 19 September 1990 (1990-09-19) <br> * page 6, line 55 - page 7, line 19; figures 1,3 * <br> * page 12, lines 24-36 * <br> * page 12, lines 38-45; figures 11a,11b * <br> Appendix 1 <br> ----- | 3,7,11 | |
| Y | GB 1 502 056 A (AMERICAN MEDICAL ELECTRONICS CORP) 22 February 1978 (1978-02-22) <br> * column 3, lines 11-20; figure 1 * <br> ----- | 4,8,12 | |

The present search report has been drawn up for all claims

| Place of search | Date of completion of the search | Examiner |
|---|---|---|
| Berlin | 15 March 2005 | Kronberger, R |

EPO FORM 1503 03.82 (P04C01)

**ANNEX TO THE EUROPEAN SEARCH REPORT
ON EUROPEAN PATENT APPLICATION NO.**

EP 04 00 7593

This annex lists the patent family members relating to the patent documents cited in the above-mentioned European search report.
The members are as contained in the European Patent Office EDP file on
The European Patent Office is in no way liable for these particulars which are merely given for the purpose of information.

15-03-2005

| Patent document cited in search report | | Publication date | Patent family member(s) | | Publication date |
|---|---|---|---|---|---|
| WO 0128414 | A | 26-04-2001 | WO 0128414 A2 | | 26-04-2001 |
| WO 9601075 | A | 18-01-1996 | DE 4423663 A1 | | 11-01-1996 |
| | | | CA 2194348 A1 | | 18-01-1996 |
| | | | CN 1159151 A | | 10-09-1997 |
| | | | WO 9601075 A1 | | 18-01-1996 |
| | | | EP 0771168 A1 | | 07-05-1997 |
| | | | JP 10503944 T | | 14-04-1998 |
| | | | KR 271095 B1 | | 01-12-2000 |
| | | | US 5924996 A | | 20-07-1999 |
| EP 1484006 | A | 08-12-2004 | JP 3566276 B1 | | 15-09-2004 |
| | | | JP 2004329542 A | | 25-11-2004 |
| | | | EP 1484006 A1 | | 08-12-2004 |
| | | | US 2004225209 A1 | | 11-11-2004 |
| US 6270455 | B1 | 07-08-2001 | US 5997476 A | | 07-12-1999 |
| | | | US 5897493 A | | 27-04-1999 |
| | | | AU 765145 B2 | | 11-09-2003 |
| | | | AU 1837900 A | | 19-06-2000 |
| | | | EP 1143854 A1 | | 17-10-2001 |
| | | | WO 0032098 A1 | | 08-06-2000 |
| | | | AU 9791098 A | | 27-04-1999 |
| | | | WO 9918532 A1 | | 15-04-1999 |
| | | | US 2003069753 A1 | | 10-04-2003 |
| | | | US 2003229514 A2 | | 11-12-2003 |
| | | | US 6334778 B1 | | 01-01-2002 |
| | | | US 6168563 B1 | | 02-01-2001 |
| | | | US 6368273 B1 | | 09-04-2002 |
| | | | US 2004019259 A1 | | 29-01-2004 |
| | | | US 6248065 B1 | | 19-06-2001 |
| | | | US 6381577 B1 | | 30-04-2002 |
| | | | US 5985559 A | | 16-11-1999 |
| | | | US 6101478 A | | 08-08-2000 |
| EP 0387630 | A | 19-09-1990 | AU 622293 B2 | | 02-04-1992 |
| | | | AU 5122190 A | | 29-11-1990 |
| | | | CA 2010165 A1 | | 13-09-1990 |
| | | | EP 0387630 A2 | | 19-09-1990 |
| | | | JP 2278142 A | | 14-11-1990 |
| | | | JP 3054739 B2 | | 19-06-2000 |
| GB 1502056 | A | 22-02-1978 | NONE | | |

EPO FORM P0459

For more details about this annex : see Official Journal of the European Patent Office, No. 12/82